(19) Europäisches Patentamt
European Patent Office
Office européen des brevets

(11) Publication number: 0 217 651
A2

(12) **EUROPEAN PATENT APPLICATION**

(21) Application number: 86307406.8

(22) Date of filing: 26.09.86

(51) Int. Cl.[4]: C 07 C 69/96
C 07 C 68/00

(30) Priority: 21.10.85 US 789809 30.09.85 US 781172
25.10.85 US 791626

(43) Date of publication of application:
08.04.87 Bulletin 87/15

(84) Designated Contracting States: DE FR GB IT SE

(71) Applicant: TEXACO DEVELOPMENT CORPORATION
2000 Westchester Avenue
White Plains New York 10650 (US)

(72) Inventor: Bhattacharya, Ajit Kumar
5 Sarah Lane
Hopewell Junction, N.Y. 12533 (US)

Nolan, John Thomas, Jr.
Relyea Terrace
Wappingers Falls, N.Y. 12590 (US)

(74) Representative: Burnside, Michael et al
Michael Burnside & Partners 2 Serjeants' Inn Fleet Street
London EC4Y 1HL (GB)

(54) **Preparation of organic carbonates.**

(57) An organic carbonate, such as dimethyl carbonate, is prepared by reacting an alcohol, such as methanol, with carbon monoxide and oxygen in the presence of a catalyst system containing a copper hydrocarbonoxy halide, such as cupric methoxychloride, Cu(OMe)Cl, and an imidazole compound, a pyridine compound or a cyclic imide.

EP 0 217 651 A2

**Description**

METHOD OF PREPARING ORGANIC CARBONATES

This invention relates to the preparation of organic carbonates. More particularly it relates to a catalyst system for the preparation of dimethyl carbonate (DMC). DMC may be used as a gasoline extender and octane enhancer, as an organic solvent, or as a reactant in place or phosgene in the preparation of isocyanates, polycarbonates, and various agricultural and pharmaceutical intermediates.

Dimethyl carbonate (DMC) may be prepared by the reaction of methanol with carbon monoxide and oxygen in the presence of a catalyst system. This invention provides an improved method of preparing organic carbonates such as dimethyl carbonate, lowers the cost of the process, substantially increases the yield and rate of production of dimethyl carbonate, and eases the separation of DMC and water from the catalyst system.

U.S. Patent 3,114,762 discloses as catalysts metal salts including chlorides and bromides of platinum and palladium plus an oxidizing agent such as iron or copper salts having the same anion.

U.S. Patent 3,227,740 discloses as catalyst mercuric halides or carboxylates.

Saegusa et al, J. Org Chem., 35, 2976-2978 (1970) discloses the reaction of CO with copper alkoxides including the dimethoxide, the diallyloxide, the chloride methoxide, and the acetylacetonate methoxide.

Romano et al IEC Prod. Res. Dev. 19, 396-403 (1980) discloses as catalyst cuprous chloride/cupric chloride methoxide.

U.S. Patent 4,218,391 discloses as catalysts salts of metals of Group IB, IIB and VIII, preferably monovalent copper such as cuprous bromide, chloride, or perchlorate.

U.S. Patent 4,318,862 discloses as catalyst salts of metals of Groups IB, IIB or VIII, typically a copper salt such as CuCl.

U.S. Patent 3,846,468 discloses as catalysts cuprous chloride complexes with various organic compounds such as pyridine, dipyridyl, imidazole, phenanthroline, alkyl, or aryl phosphines, dimethyl sulfoxide, dimethyl formamide, quinuclidine, acetonitrile, benzonitrile, malonitrile, succinodinitrile, or adiponitrile.

U.S. Patent 3,980,690 discloses as catalyst a complex of copper chloride and poly-4-cinylpyridine.

Rivetti et al, J. Organometallic Chem, 174 (1979) 221-226 discloses as catalysts palladium (II) complexes in the presence of ligands and added bases. Alkyl phosphines are said to inhibit carbonylation almost completely. The presence of tertiary amines enhances the formation of dimethyl carbonate. Low yields (6% or less) of dimethyl carbonate are obtained with $Pd(OAc)_2$ in the presence of ligands such as $R_3P$ where R is $p\text{-}C_6H_4OCH_3$. Yield is increased to 61% in the presence of a base such as diisopropylethylamine.

U.S. Patent 3,952,045 discloses as catalysts organic phosphorus compounds such as phosphine oxide, phosphite, phosphate, or phosphonate plus copper halides.

U.S. Patent 4,360,477 discloses as catalysts cupric halides inter alia.

Yang et al CA86, 171868u (1977) discloses as catalysts $PdCl_2$, $CuCl_2$, $MnCl_2$, and LiCl.

Lapidus et al CA93, 72338j (1980) discloses as catalyst $MnCl_2$, $KMnO_4$, $CuCl_2$, LiCl, and $Mn(OAc)_3$.

Itatani, Japanese patent publication 54-24827 pub 24 Feb. 1979 discloses as catalyst a cuprous halide plus as auxiliary catalyst a halide of an alkali metal or an alkaline earth metal.

U.S. Patent 4,370,275 discloses as catalyst compositions containing copper, chemically bonded oxygen, and halogen and a nitrogen base. A typical catalyst contains CuO or $Cu(OCl)_2$ and n-butylamine inter alia. Preferred combinations include: $CuCO_3$, $Cu(OH)_2$, $CuCl_2$ and pyridine hydrochloride etc.

U.S. Patent 4,131,521 discloses an electromechanical process utilizing a non-fluoride halide-containing electrolyte.

U.S. Patent 4,113,762 discloses as catalyst a complex of copper (as CuCl) with $VCl_3$, $CrCl_3$, $FeCl_3$, $CoCl_2$, $AlCl_3$, or $SiCl_4$.

U.S. Patent 4,361,519 discloses as catalyst (i) a Bronsted base such as a quaternary ammonium, phosphonium or sulfonium compound or an alkoxide or hydroxide or alkali metal or alkaline earth metal or a salt of a strong base and a weak acid or amines etc. plus (ii) a Group VIII B element Ru, Rh, Pd, Os, Ir or Pt plus (iii) oxygen plus (iv) a redox catalyst such as a Mn or Co containing catalyst. A typical system includes (i) a pentamethylpiperidine, (ii) $PdBr_2$ and (iii) pyridine adduct or salicylaldehyde - ethylene diamine Co (II) complex.

European patent 0,071,286 discloses as catalyst a copper compound such as a halide (in the presence of an amine) plus a sulphone such as dimethyl sulphone or sulfolane.

The invention is directed to a method of preparing an organic carbonate comprising reacting a monohydric alcohol with carbon monoxide and oxygen in the presence of a catalyst system containing a copper hydrocarbonoxy halide and recovering the product, characterized in that the catalyst system includes an imidazole compound, a pyridine compound or a cyclic amide.

The method comprises reacting the alcohol with carbon monoxide and oxygen in the presence of a catalyst system containing

(a) as a catalyst, a copper hydrocarbonoxy halide Cu(OR")X, wherein R" is a hydrocarbon group selected from the group consisting of alkyl, alkaryl, aralkyl, cycloalkyl, and aryl and X is a halide; and

(b) an imidazole compound, a pyridine compound or a cyclic amide.

The presence of the added imidazole compound, pyridine compound or cyclic amide substantially increases

the rate of production of the organic carbonate.

The charge alcohol which may be employed in practice of the method of this invention may include those characterized by the formula R'OH.

In the above compound, R' may be a hydrocarbon group selected from the group consisting of alkyl, aralkyl, cycloalkyl, aryl, and alkaryl, including such radicals when inertly substituted. When R' is alkyl, it may typically be methyl, ethyl, n-propyl, iso-propyl, n-butyl, i-butyl, sec-butyl, amyl, octyl, decyl, octadecyl, etc. When R' is aralkyl, it may typically be benzyl, betaphenylethyl, etc. When R' is cycloalkyl, it may typically be cyclohexyl, cycloheptyl, cyclooctyl, 2-methylcycloheptyl, 3-butylcyclohexyl, 3-methylcyclohexyl, etc. When R' is aryl, it may typically be phenyl, naphthyl, etc. When R' is alkaryl, it may typically be tolyl, xylyl, etc. R' may be inertly substituted, i.e. it may bear a non-reactive substituent such as alkyl, aryl, cycloalkyl, ether, etc. Typically inertly substituted R' groups may include 2-ethoxyethyl, carboethoxymethyl, 4-methyl cyclohexyl, etc. The preferred R' groups may be lower alkyl, i.e. $C_1$-$C_{10}$ alkyl, groups including methyl, ethyl, n-propyl, i-propyl, butyls, amyls, hexyls, octyls, decyls, etc. R' may preferably be methyl.

The charge alcohol may be a phenol i.e. when R' is aryl. The notation R'OH is intended to include polyols such as ethylene glycol, glycerine, sorbitol, poly (oxyalkylene) polyols, etc; in these latter compounds, the formula may more typically be represented as R' $(OH)_n$wherein R' is derived from an alkyl group and n is an integer, typically 2-10.

The charge alcohols which may be employed include these listed below in Table I.

TABLE I

methanol
ethanol
n-propanol
i-propanol
benzyl alcohol
phenol
ethylene glycol
glycerine
sorbitol
poly(oxyethylene-10) glycol

The preferred alcohols are the lower ($C_1$-$C_4$) alkanols; and most preferred is methanol.

The carbon monoxide charge which may be employed may be a pure gas. More commonly it may be a synthesis gas of high purity from which most of the hydrogen and carbon dioxide have been removed.

The catalyst, i.e. copper hydrocarbonoxy halide Cu(OR")X may be one wherein X is fluorine, chlorine, bromine, or iodine. Preferably X is chlorine or bromine and more preferably chlorine.

R" may be selected from the same group as R'; and preferably R" is a lower alkyl i.e. $C_1$-$C_{10}$ alkyl. Preferably R" is methyl. Typical compounds may include those listed below in Table II. The preferred catalyst being the first listed, i.e. cupric methoxychloride.

TABLE II

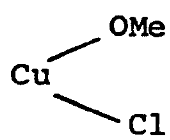

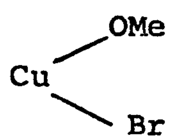

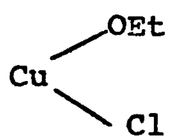

The copper salt catalyst system containing Cu(II) (OMe)Cl/Cu(I)Cl is only sparingly soluble in methanol and the rate of DMC formation is undesirably low. This invention provides a large increase in the yield and rate of organic carbonate formation by the addition of the imidazole compound, pyridine compound or cyclic amide. According to the present invention, it has been found that the rate of DMC formation in the presence of these additives is several times higher than that under similar conditions in either triethylamine or dimethylaminobenzene. An additional advantage of the additives is that they are not volatile and remain with the catalyst during

any subsequent separation of the reaction mixture by any flash or distillation method.

The imidazole compounds have the formula:

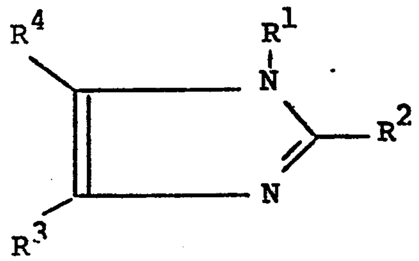

wherein $R^1$ is H, R, OR, COOR, or $NR^5R^6$; and $R^2$, $R^3$, and $R^4$ are each H, F, Cl, Br, I, $NO_2$, R, OR, COOR, CN, or $NR^5R^6$ where R is a ($C_1$-$C_{10}$) alkyl, aralkyl, cycloalkyl, aryl or alkaryl group, and $R^5$ and $R^6$ are each a ($C_1$-$C_8$) alkyl group, or $R^5$ and $R^6$ combined are ($C_3$-$C_8$) alkylene or oxa-, thia, or aza-alkylene groups, and where said imidazole ring is mono, di, tri, or tetra substituted; or $R^1$ and $R^2$, $R^1$ and $R^4$, or $R^3$ and $R^4$ combined from a cyclic, heterocyclic or aromatic group.

The imidazole compounds, according to the present invention may include imidazole, 1-methylimidazole, 1-methyl-2-ethyl-imidazole, 4,5-dimethylimidazole, 4,5-cyclohexanoimidazole or benzimidazole.

Also, according to the above formula, the imidazole compounds may be represented by the following formulas: imidazole (where $R^1$, $R^2$, $R^3$ and $R^4$ are each H).

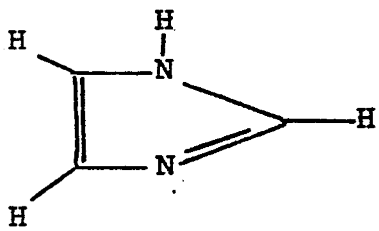

1.

1-methylimidazole (where $R^1$ is $CH_3$; $R^2$, $R^3$ and $R^4$ are each H)

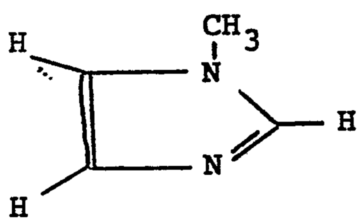

2.

1-methyl-2-ethylimidazole (where $R^1$ is $CH_3$; $R^2$ is $C_2H_5$; $R^3$ and $R^4$ are each H)

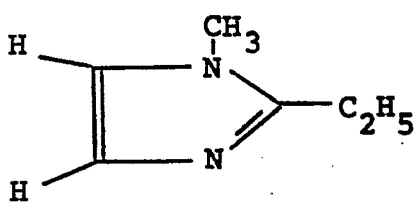

3.

4-5, dimethylimidazole (where $R^1$ and $R^2$ are each H; $R^3$ and $R^4$ are each $CH_3$)

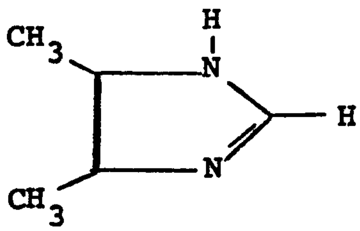

4.

4,5-cyclohexanoimidazole (where $R^1$ and $R^2$ are each H; $R^3$ and $R^4$ combined form cyclohexano moiety)

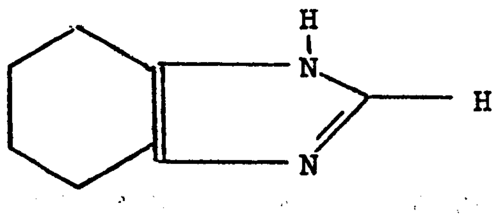

5.

benzimidazole (where $R^1$ and $R^2$ are each H; $R^3$ and $R^4$ combined form benzene ring).

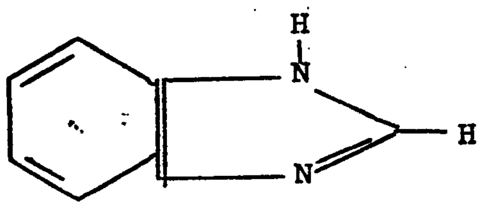

6.

The pyridine compounds have the formula

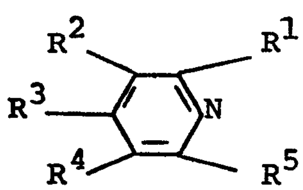

wherein $R^1$, $R^2$, $R^3$, $R^4$ and $R^5$ may each be H, F, Cl, Br, I, $NO_2$, R, OR, SR, CN, COOR, or $NR^6R^7$ where R is a ($C_1$-$C_{10}$) alkyl, aralkyl, cycloalkyl, aryl or alkaryl group; $R^6$ and $R^7$ are each a ($C_1$-$C_8$) alkyl group, or $R^6$ and $R^7$ combined are ($C_3$-$C_8$) alkylene or oxa, thia-, or aza-alkylene groups, and where said pyridine ring is mono, di, tri, tetra, or penta substituted; and recovering the organic carbonate product.

The pyridine compounds, according to the present invention may include 4-dimethylaminopyridine, 2-diethylaminopyridine, methyl isonicotinate, N,N-dimethylnicotinamide, 4-methoxypyridine, 2-(methylthio) pyridine, 4-tertiary-butylpyridine, pyridine, 4-(morpholino)pyridine, 4-(N-methylpiperazino)pyridine, 2,2'-dipyridyl, or 2-pyridyl-2'pyrimidyl.

Also according to the above formula, the pyridine compounds may be presented by the following formulas:

4-dimethylaminopyridine [where $R^3$ is $N(CH_3)_2$]

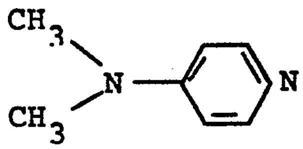

1.

2-diethylaminopyridine [where $R^1$ is $N(C_2H_5)_2$]

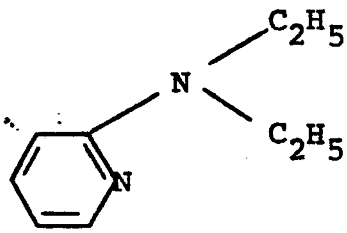

2.

methyl isonicotinate [where $R^3$ is $(COOCH_3)$]

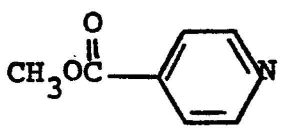

3.

N,N-dimethylnicotinamide [where $R^2$ is $CON(CH_3)_2$]

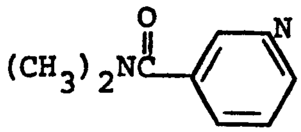

4.

4-methoxypyridine [where $R^3$ is $(CH_3O)$]

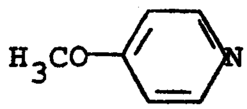

5.

2-(methylthio)pyridine [where $R^1$ is $(SCH_3)$]

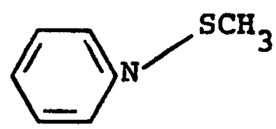

6.

4-tertiary-butylpyridine [where $R^3$ is $C(CH_3)_3$]

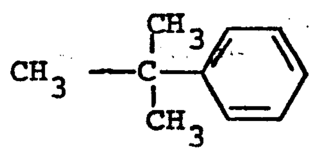

7.

pyridine (where $R^3$ is H)

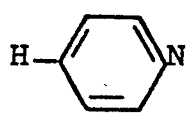

8.

4-(morpholino)pyridine [where $R^3$ is 4-(morpholino)]

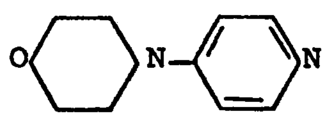

9.

4-(N-methylpiperazino)pyridine [where $R^3$ is 4-(N-methylpiperazino)]

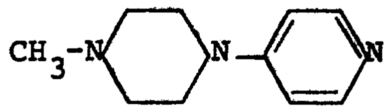

10.

2,2'-dipyridyl [where $R^1$ is 2'-pyridono]

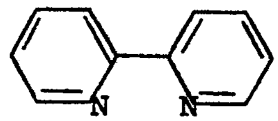

11.

2-pyridyl-2'-pyrimidyl [where $R^1$ is 2'-pyrimidyl]

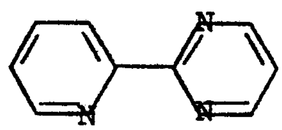

12.

2,3-dimethylpyridine [where $R^1$ and $R^2$ are each $CH_3$]

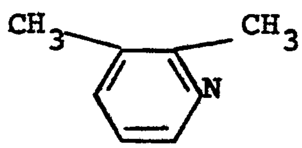

13.

pentachloropyridine [where $R^1$, $R^2$, $R^3$, $R^4$ and $R^5$ are each Cl]

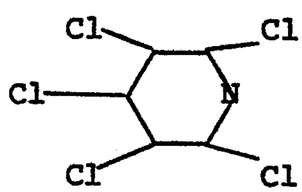

14.

In another embodiment of the present invention, there are other pyridine and pyrimidine derived compounds, which may be used. These include the heterocyclic compounds provided below in Table III.

TABLE III

2,2'-dipyrimidyl

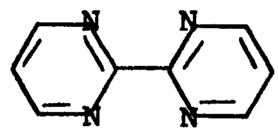

15.

1,10-Phenanthroline

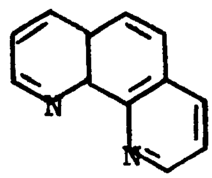

16.

Quinoline

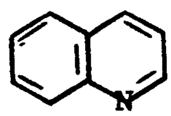

17.

Isoquinoline

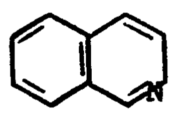

18.

The cyclic amide has the formula:

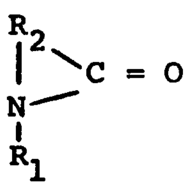

where $R_1$ is H or a ($C_1$-$C_8$) alkyl group and $R_2$ is a ($C_3$-$C_8$) alkylene group.

The cyclic amide, according to the present invention may be 2-pyrrolidinone, N-methyl-2-pyrrolidinone, N-methyl-2-piperidone, ε-Caprolactam, N-methylcaprolactam, or 2-azacyclononanone.

Also, according to the above formula, the cyclic amides may be represented by the following formulas:

2-pyrrolidone (wherein $R_1$ is H and $R_2$ is propylene)

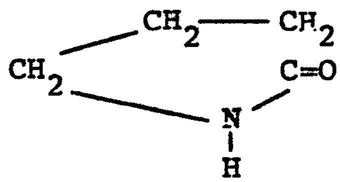 ; and 1.

N-methyl-2-pyrrolidone (wherein $R_1$ is $CH_3$ and $R_2$ is propylene)

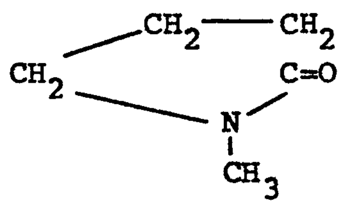 ; and 2.

N-methyl-2-piperidone (wherein $R_1$ is $CH_3$ and $R_2$ is tetramethylene)

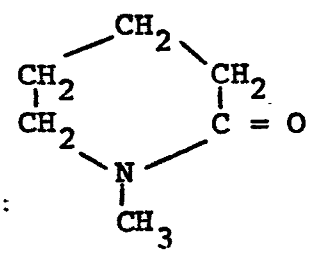 3.

ε-Caprolactam (where $R_1$ is H and $R_2$ is pentamethylene)

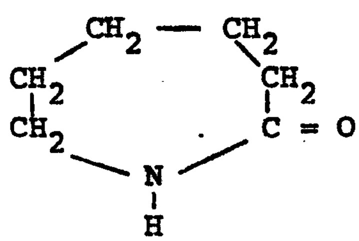 4.

N-methylcaprolactam (wherein $R_1$ is $CH_3$ and $R_2$ is pentamethylene)

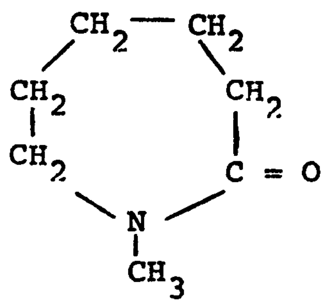

5.

2-azacyclononanone (wherein $R_1$ is H, and $R_2$ is heptamethylene)

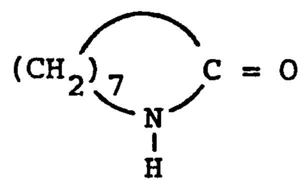

6.

The catalyst system may be present in the reaction mixture in an amount of about 0.1 to about 50 parts, preferably about 0.1 to about 20 parts, and more preferably about 10 parts per 100 parts of charge methanol.

The practice of the method of this invention may be carried out by adding 100 parts of an optimum mixture of the alcohol ROH, such as methanol and the imidazole compound, pyridine compound or cyclic amide to the reaction mixture. The weight percent (wt.%) of additive in methanol may vary from 1 to 95%, preferably about 50%, and more preferably about 75%. The catalyst system may then be added. The system is then subjected to inert gas typically nitrogen at a partial pressure of 34-6900 kPa, preferably 690-2070 kPa, say about 690 kPa and heated to 20° C-170° C preferably 80-120° C, say about 90° C at a total pressure of 69-14000 kPa, preferably 1030-4100 kPa, say about 1030 kPa over 0.25-2 hours, say about 0.5 hour.

Carbon monoxide-containing gas is then admitted to a carbon monoxide partial pressure of 34-20700 kPa, preferably 690-6200 kPa, say 2400 kPa over 0.25-10 hours, say 1 hour.

During this period, the following reaction occurs in the preferred embodiment:

$2Cu(OMe)Cl + CO \rightarrow (MeO)_2CO + 2CuCl$

At the end of this time, the reaction mixture may be rapidly cooled to 20°C-90°C, say 25°C at a total pressure of 103-20700 kPa, say 2400 kPa.

The reaction mixture may be flashed under reduced pressure to remove gases, methanol, DMC and water or depressured and then distilled to azeotropically distill off various fractions containing methanol, dimethyl carbonate and water. The product may be further treated to effect greater purification of the impure dimethyl carbonate.

The residual catalyst system (0.1-50 parts, preferably 10 parts) may be regenerated by contacting with an oxygen-containing gas, typically air at 20°C-65°C, say 45°C for 1-50 hours, say 6 hours in the presence of excess alcohol, typically methanol in an amount of 100 parts.

During this regeneration step, the following reaction occurs in the preferred embodiment:

$2CuCl + 2MeOH + {}^1/_2O_2 \rightarrow$

$2Cu(OMe)Cl + H_2O$

At the end of the regeneration period, the catalyst in methanol may be recycled if the water content is less than about 1.0 wt.%. If more water than this is present, the catalyst may be separated and then dried by heating to 30°C-60°C, preferably to 40°C (under reduced pressure) for 1-10 hours, preferably for 6 hours to yield a substantially anhydrous catalyst system which is recycled using anhydrous methanol.

Practice of the method of this invention will be apparent to those skilled in the art from the following examples, wherein as elsewhere in this specification, all parts are parts by weight unless otherwise noted.

EXAMPLES I-VI

In these examples there was added to the reaction vessel 190 ml of anhydrous methanol and 18.2 g (0.14 mol) of anhydrous Cu(OMe)Cl and 0.07 mole of an organic nitrogen base compound including the imidazole ligands of this invention (i.e. triethylamine, dimethylaminobenzene N-methylimidazole, imidazole). In Examples I and II, no such ligand was present.

In each of the examples, the procedure and conditions were the same except that the nitrogen base compound was different.

The reaction mixture was pressurized to 690 kPa with nitrogen, heated to 90° C and maintained at 90° C for 0.5 hour. The pressure was increased to 3400 kPa with carbon monoxide and the stirring was continued. The reaction mixture was then cooled to room temperature, and depressurized. The reaction mixture was distilled with added methanol (200 ml) to recover azeotrope containing methanol and dimethyl carbonate. Analysis by Gas Chromatography indicated the yields (based on copper salt) shown below in Table IV.

Methanol (100 ml) was added and the catalyst was regenerated by bubbling air through the suspension at 45° C for 6 hours. The regenerated catalyst in this reaction mixture was recycled for reaction with CO under standard reaction conditions as described above. The product was recovered and analyzed for DMC as mentioned above. The numbers in parentheses represent the yield attained in a subsequent run in which the catalyst used has been regenerated, the reaction conditions being otherwise the same.

## TABLE IV

### Production of DMC

| Example | Nitrogen Base (B) | B/[Cu][1] Mol Ratio | % Yield of DMC (2,3,4) |
|---|---|---|---|
| I | None | 0.0 | 8(14) |
| II | None | 0.0 | 19(22)[5] |
| III | Dimethylaminobenzene | 0.5 | 4 |
| IV | Triethylamine | 0.5 | 3(4) |
| V | N-methylimidazole | 0.5 | 72 |
| VI | Imidazole | 0.5 | 101 |

[1] [Cu] = cupric methoxychloride (18.2g, 0.14 mol)/190 ml methanol

[2] Reaction conditions: 90°C/3400 kPa $N_2$:CO (3:7)/15 min.

[3] Gas chromatographic analyses were employed to determine the yield based on copper salt added and 100% DMC product selectivity.

[4] The numbers in parentheses represent the yield attained in a subsequent run in which the catalyst used has been regenerated, the reaction conditions being otherwise the same.

[5] Reaction conditions were the same as in footnote 2 excepting that the reaction time was 30 minutes.

As shown in Table IV above, the addition of an imidazole ligand of the present invention greatly increased the yield of dimethyl carbonate (DMC). In fact, the yield was more than 9 to 12 times the yield when methanol was used alone. It is apparent that the process of this invention makes it possible to obtain higher yields of DMC in shorter time, i.e. to increase the rate of formation of desired dimethyl carbonate.

EXAMPLES VII-XIX

In these examples there was added to the reaction vessel 190 ml of anhydrous methanol and 18.2 g (0.14 mol) of anhydrous Cu(OMe)Cl and 0.07 mole of an organic nitrogen base compound including the pyridine ligands of this invention, (i.e. triethylamine, pyridine, 4-dimethylaminopyridine, methyl isonicotinate, hexamethylmelamine, 4-*tert*-butylpyridine, 4,4'-dpyridyl and 2,2'-dipyridyl). In Examples VII and VIII, no such

ligand was present, and in Example XII 0.14 mole of pyridine was employed.

In each of the examples, the procedure and conditions were the same except that the nitrogen base compound was different.

The reaction mixture was pressurized to 690 kPa with nitrogen, heated to 90°C and maintained at 90°C for 0.5 hour. The pressure was increased to 3400 kPa with carbon monoxide and the stirring was continued. The reaction mixture was then cooled to room temperature, and depressurized. The reaction mixture was distilled with added methanol (200 ml) to recover azeotrope containing methanol and dimethyl carbonate. Analysis by Gas Chromatography indicated the yields based on copper salt shown below in Table V.

Methanol (100 ml) was added and the catalyst was regenerated by bubbling air through the suspension at 45°C for 6 hours. The regenerated catalyst in this reaction mixture was recycled for reaction with CO under standard reaction conditions as described above. The product was recovered and analyzed for DMC as mentioned above. The numbers in parentheses represent the yield attained in a subsequent run in which the catalyst used has been regenerated, the reaction conditions being otherwise the same.

## TABLE V

### Production of DMC

| Example | Nitrogen Base (B) | B/[Cu][1] Mol Ratio | % Yield of DMC (2,3,4) |
|---|---|---|---|
| VII | None | 0.0 | 8(14) |
| VIII | None | 0.0 | 19(22)[5] |
| IX | Dimethylaminobenzene | 0.5 | 4 |
| X | Triethylamine | 0.5 | 3(4) |
| XI | Pyridine | 0.5 | 51(70) |
| XII | Pyridine | 1.0 | 90[5] |
| XIII | 4-dimethylaminopyridine | 0.5 | 76 |
| XIV | 4-dimethylaminopyridine | 0.5 | 97[5] |
| XV | Methyl isonicotinate | 0.5 | 42(66)[5] |
| XVI | Hexamethylmelamine | 0.5 | 19[5] |
| XVII | 4-*tert*-butylpyridine | 0.5 | 46 |
| XVIII | 4,4'-dipyridyl | 0.5 | 2 |
| XIX | 2,2'-dipyridyl | 0.5 | 74 |

[1] [Cu] = cupric methoxychloride (18.2 g, 0.14 mol)/190 ml methanol.

[2] Reaction conditions: 90°C/3400 kPa $N_2$:CO (3:7)/15 min.

[3] Gas chromatographic analyses were employed to determine the yield based on copper salt added and 100% DMC product selectivity.

[4] The numbers in parentheses represent the yield attained in a subsequent run in which the catalyst used has been regenerated, the reaction conditions being otherwise the same.

[5] Reaction conditions were the same as in footnote 2 excepting that the reaction time was 30 minutes.

As shown in Table V above, the addition of a pyridine ligand of the present invention greatly increased the yield of dimethyl carbonate (DMC). In fact, the yield was more than 3 to 9 times the yield when methanol was

used alone. It is apparent that the process of this invention makes it possible to obtain higher yields of DMC in shorter time, i.e. to increase the rate of formation of desired dimethyl carbonate.

EXAMPLES XX-XXIV

In these examples there was added to the reaction vessel 141 ml of anhydrous methanol and 18.2 g (0.14 mol) of anhydrous Cu(OMe)Cl and 47 ml of cosolvent (i.e. acetonitrile, triglyme, dimethyl phthalate, 2-pyrrolidinone or N-methyl-2-pyrrolidinone). The methanol and cyclic amide cosolvent were added in ratio of methanol to cosolvent, of about 3:1.

In each of the examples, the procedure and conditions were the same except that the cosolvent was different.

The reaction mixture was pressurized to 690 kPa with nitrogen, heated to 90°C and maintained at 90°C for 0.5 hour. The pressure was increased to 3400 kPa with carbon monoxide and the stirring was continued for 0.25 hour. The reaction mixture was then cooled to room temperature, and depressurized. The reaction mixture was distilled with added methanol (200 ml) to recover azeotrope containing methanol and dimethyl carbonate. Analysis by gas chromatography for each run indicated the yields based on copper salt shown below in Table VI.

Methanol (100 ml) was added and the catalyst was regenerated by bubbling air through the suspension at 45°C for 6 hours. The regenerated catalyst in this reaction mixture was recycled for reaction with CO under standard reaction conditions as described above. The product was recovered and analyzed for DMC as mentioned above. The numbers in parentheses represent the yield attained in a subsequent run in which the catalyst used has been regenerated, the reaction conditions being otherwise the same.

TABLE VI

Production of DMC

| Example | Cosolvent | B.P.(%) of Cosolvent | Yield (%)[1] |
|---|---|---|---|
| R[2] | Methanol (neat) | 65 | 8(14) |
| XX | Acetonitrile | 81 | 4(5) |
| XXI . | Triglyme | 216 | 6(9) |
| XXII | Dimethyl phthalate | 282 | 9(8) |
| XXIII | 2-pyrrolidinone | 245 | 83 |
| XXIV | N-methyl-2-pyrrolidinone | 202 | 87 |

[1]Gas chromatographic analysis using a Porapak P (100-120 mesh) column was employed to determine the yield based on copper salt added and 100% DMC product selectivity.

[2]Reference cosolvent which was compared with present cosolvents.

As shown in Table VI above, the addition of a cosolvent of the present invention greatly increased the yield of dimethyl carbonate (DMC). In fact, the yield was more than 6 to 10 times the yield when methanol was used. It is apparent that the process of this invention makes it possible to obtain higher yields of DMC in shorter times, i.e. to increase the rate of formation of desired DMC.

**Claims**

1. A method of preparing an organic carbonate comprising reacting a monohydric alcohol with carbon monoxide and oxygen in the presence of a catalyst system containing a copper hydrocarbonoxy halide and recovering the product, characterised in that the catalyst system includes an imidazole compound, a pyridine compound or a cyclic amide.

2. A method of preparing an organic carbonate according to claim 1, characterised in that the monohydric alcohol has the formula R'OH in which R' may be an alkyl, aralkyl, cycloalkyl, aryl, or aryl group.

3. A method of preparing an organic carbonate according to claim 1, characterised in that the copper hydrocarbonoxy halide has the formula Cu(OR")X wherein X is a halogen atom and R" is an alkyl. aralkyl, cycloalkyl, aryl, or aryl group.

4. A method of preparing an organic carbonate according to claim 1, characterised in that the monohydric alcohol is methanol and the copper hydrocarbonoxy halide is cupric methoxy chloride.

5. A method of preparing an organic carbonate according to claim 4, characterized in that the reaction is carried out at a temperature from 20° to 170° C and at a pressure from 69 to 13800 kPa for a period from 0.25 to 2 hours.

6. A method of preparing an organic carbonate according to claim 5, characterised in that the reaction is carried out at a temperature from 80° to 120° C and at a pressure from 1030 to 4140 kPa for a period of 0.5 hours.

7. A method of preparing an organic carbonate according to claims 4, 5 or 6, characterised in that from 0.1 to 50 parts of catalyst are present per 100 parts of methanol.

8. A method of preparing dimethyl carbonate according to any of the claims 4 to 7, characterised in that the imidazole compound has the formula:

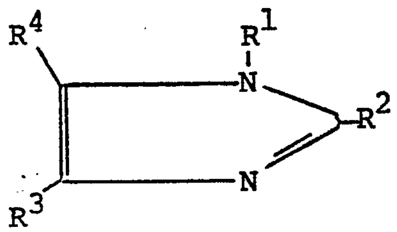

wherein $R^1$ is H, R, OR, COOR, or $NR^5R^6$; and $R^2$ , $R^3$ and $R^4$ are each H, F, Cl, Br, I, $NO_2$, R, OR, SR, COOR, CN, or $NR^5R^6$ where R is a ($C_1$-$C_{10}$) alkyl, aralkyl, cycloalkyl, aryl or alkaryl group, and $R^5$ and $R^6$ are each a ($C_1$-$C_8$) alkyl group, or $R^5$ and $R^6$ combined are ($C_3$-$C_8$) alkylene or oxa-, thia, or aza-alkylene groups, and where said imidazole ring is mono, di, tri, or tetra substituted; or $R^1$ and $R^2$, $R^1$ and $R^4$, or $R^3$ and $R^4$ combined form a cyclic, heterocyclic or aramatic group.

9. A method of preparing an organic carbonate according to claim 8, characterized in that the imidazole compound is one of the imidazole compounds numbered 1 to 6 herein.

10. A method of preparing an organic carbonate according to any of the claims 4 to 7, characterised in that the pyridine compound has the formula:

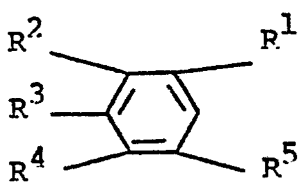

wherein $R^1$, $R^2$, $R^3$, $R^4$, and $R^5$ may each be H, F, Cl, Br, I, $NO_2$, R, OR, SR, CN, COOR, or $NR^6R^7$ where R is a ($C_1$-$C_{10}$) alkyl, aralkyl, cycloalkyl, aryl or alkaryl group; $R^6$ and $R^7$ are each a ($C_1$-$C_8$) alkyl group, or $R^6$ and $R^7$ combined are ($C_3$-$C_8$) alkylene or oxa, thia, or azaalkylene groups, and where said pyridine ring is mono, di, tri, tetra, or penta substituted; and recovering the organic carbonate product.

11. A method of preparing a organic carbonate according to claim 10, characterised in that the pyridine compound is one of the pyridine compounds numbered 1 to 18 herein.

12. A method of preparing an organic carbonate according to any of the claims 4 to 7, characterised in that the cyclic amide has the formula:

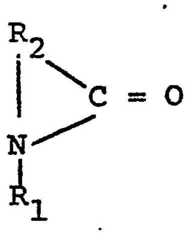

wherein $R_1$ is H or a ($C_1$-$C_8$) alkyl group and $R_2$ is a ($C_3$-$C_8$) alkylene group.

13. A method of preparing an organic carbonate according to claim 12, characterised in that the cyclic amide is one of the cyclic amides numbered 1 to 6 herein.

14. Organic carbonates prepared by the method of any of the claims 1 to 13.